# EUROPÄISCHE PATENTSCHRIFT

(11) **EP 3 574 887 B1**
(45) Veröffentlichungstag und Bekanntmachung des Hinweises auf die Patenterteilung: **16.09.2020**
(21) Anmeldenummer: 19176915.7
(22) Anmeldetag: 28.05.2019
(51) Int. Cl.: A61K 6/18, A61K 6/898

(54) **ADSTRINGIERENDE ALUMINIUMFREIE RETRAKTIONSPASTE FÜR DEN PROFESSIONELLEN ZAHNÄRZTLICHEN GEBRAUCH**
ASTRINGENT ALUMINUM-FREE RETRACTION PASTE FOR PROFESSIONAL DENTAL USE
PÂTE DE RÉTRACTION ASTRINGENTE SANS ALUMINIUM DESTINÉE À L'UTILISATION DENTAIRE PROFESSIONNELLE

(30) Priorität: 30.05.2018 DE 102018112996
(43) Veröffentlichungstag der Anmeldung: 04.12.2019
(73) Patentinhaber: VOCO GmbH, 27472 Cuxhaven (DE)
(72) Erfinder: Wolfkühler, Denis, 21762 Otterndorf (DE); Plaumann, Manfred Thomas, 27472 Cuxhaven Niedersachsen (DE)

(56) Entgegenhaltungen:
- EP-A1- 2 070 505
- EP-A2- 0 329 098
- DE-A1-102010 021 085
- US-A1- 2005 008 583
- M.S. Rodriguez ET AL: "Relationship Between Astringency and Chitosan-Saliva Solutions Turbidity at Different pH", Journal of Food Science, vol. 68, no. 2, 1 March 2003 (2003-03-01), pages 665-667, XP055676544, US ISSN: 0022-1147, DOI: 10.1111/j.1365-2621.2003.tb05728.x

## Beschreibung

Die vorliegende Erfindung betrifft eine adstringierende aluminiumfreie Retraktionspaste, die von Zahnärzten vor einer Abformung zur Freilegung des Präparationsrandes verwendet werden kann, umfassend Chitosan bzw. Chitosanderivate, Weinsäure, Lösungsmittel, vorzugsweise Wasser und/oder Alkohole sowie Füllstoffe für den professionellen zahnärztlichen Gebrauch. Die Erfindung umfasst ferner eine adstringierende Retraktionspaste zur Anwendung in einem therapeutischen Verfahren zur Restauration eines oder mehrerer Zähne sowie ein entsprechendes Kit.

Für die Herstellung indirekter Restaurationen in der Zahnmedizin, wie beispielsweise Kronen oder Brücken, ist es notwendig, am präparierten Zahn eine Präzisionsabformung durchzuführen. Um beim Abdruck auch den subgingivalen Bereich des Zahnes zu erfassen, muss vor diesem Arbeitsschritt der Abformung zunächst der Präparationsrand freigelegt werden.

Hierzu kann man einen Retraktionsfaden benutzen, der in den Sulcus gingivae gelegt wird. Da es sehr wichtig ist, bei der Abformung einen trockenen Präparationsrand zu haben, werden die Fäden in der Regel in eine adstringierende chemische Lösung getränkt. Sehr oft werden hierfür aluminiumhaltige Lösungen benutzt, allerdings haben diese Lösungen den Nachteil, dass sie gesundheitsschädlich sind. Laut der Stellungnahme Nr. 007/2014 des Bundesinstitut für Risikobewertung wurde wissenschaftlich erwiesen, dass "hohe Aluminiumdosen neurotoxische Wirkung beim Menschen" zeigen. Außerdem sei die, durch die Behörde für Lebensmittelsicherheit festgesetzte tolerierbare wöchentliche Aufnahmemenge von Aluminium bei einem Teil der Bevölkerung schon durch den Konsum von Lebensmittel erreicht und sollte somit nicht noch durch andere Produkte erhöht werden.

Diese Technik der Retraktionsfäden hat auch noch weitere Nachteile. Da der Retraktionsfaden durch Druck eingesetzt wird, ist es schwierig, präzise zu arbeiten, denn man kann den ausgeübten Druck und somit die Verschiebung des Zahnfleisches nicht genau bestimmen und dies kann dazu führen, dass zu viel Gingiva verschoben wird. Wenn zu viel Druck ausgeübt wird, kann es sogar passieren, dass sich die Gingiva nach dem Entfernen des Retraktionsfadens nicht wieder zurückbildet. Diese Technik ist außerdem auch sehr zeitaufwendig.

Eine bessere Methode besteht in der Nutzung einer Retraktionszusammensetzung, da man mit einer Retraktionszusammensetzung schonender die Gingiva verschieben kann. Hierbei wird die Zusammensetzung, die in der Regel adstringierende Mittel beinhaltet, in den Sulcus gingivae appliziert und kann die Gingiva schonender verschieben.

Retraktionszusammensetzungen sind aus dem Stand der Technik bekannt.

WO 2014/100305 A1 beschreibt ein zweikomponentiges, pastöses Retraktionssystem, bestehend aus klassischen, additionsvernetzenden Silikonen, die ein adstringierendes Mittel, einen nicht verstärkenden Füllstoff und einen verstärkenden Füllstoff umfassen. Durch den Prozess der Hydrosilylierung bewirkt der sich entwickelnde Wasserstoff eine Schaumbildung während der Härtung und expandiert die Masse. Als adstringierendes Mittel werden Eisensulfat, Eisenchlorid und Mischungen dieser Salze, Permanganate, Zinkchlorid oder ein wasserlösliches adstringierendes Aluminiumsalz wie Kaliumaluminiumsulfat, Aluminiumammoniumsulfat, Aluminiumsulfat, Aluminiumchlorhydrate, Aluminiumacetat oder deren Mischungen offenbart. In einer Ausführungsform des zweikomponentigen, pastösen Retraktionssystems sind die adstringierenden Mittel verkapselt.

US 2008/0220050 A1 beansprucht eine viskose Retraktionspaste, die aus Tonerde, Glasfüllstoff, einem Adstringens und Wasser zusammengesetzt ist. Als adstringierendes Mittel werden Alaun (Kaliumaluminiumsulfat), Aluminiumchlorid, Aluminiumsulfat, Eisenchlorid, Eisensulfat, Zinkchlorid, Zinksulfat, Adrenalin sowie deren Mischungen angegeben.

Aus der US 2011/0046262 A1 ist ein pastöses Retraktionsmaterial, umfassend ein pastenbildendes Material, superabsorbierende Partikel und mindestens ein Adstringens bekannt, welches nach dem Applizieren durch die Aufnahme von Flüssigkeit sein Volumen erhöht. Als pastenbildendes Material werden Polysiloxane, Polyalkylenoxide, multifunktionelle Alkohole, Ester oder Halbester dieser Alkohole mit gesättigten und/oder ungesättigten Carbonsäuren, Wasser sowie Kombinationen dieser Verbindungen beansprucht. In Anspruch 12 werden unter anderem als Adstringens die folgenden Stoffe benannt: wasserlösliche Aluminiumsalze, Eisensalze, Zinksalze, Alkalihalogenide, Aluminiumacetat, Adrenalin, Noradrenalin, Oxichinolin, Gerbsäure, Tannin, Hydroperoxide, Carbamidperoxide, Peroxosalze, Calciumsalze oder eine Kombination dieser Verbindungen und besonders bevorzugt Kaliumalaun, Aluminiumchlorid, Aluminiumchlorat, Dialuminiumpentahydroxychlorid, Aluminiumhydroxychlorid, Aluminiumsulfat, Aluminiumoxalat, Aluminiumacetat, Aluminiumlactat, Aluminiumtrifluoromethansulfonat, Aluminiumnitrat, Aluminiumammoniumsulfat sowie Mangan- oder Wismutsalze wie Kaliumpermanganat, Wismutnitrat oder Wismutsalicylat und andere basische, saure oder amphotere wasserlösliche Aluminiumsalze, Zinkchlorid, Zinkoxid, Zinksalicylat, Zinkstearat, Zinksulfat, Zinkphosphat, Zinkhexafluorosilicat, Zinkphenolsulfonat, Eisenchlorid, Eisensulfat, Natriumchlorid, Calciumsulfat, Calciumsulfatdihydrat, Calciumsulfathemihydrat, Calciumhydroxid, Calciumhydrogenphosphat, Calciumlactat, Calciumgluconat und Calciumacetat, Calciumsalze aus Aminosäuren, Aldonsäuren und Uronsäuren oder die Kombinationen aus zweien oder mehreren dieser Verbindungen.

US 2012/0077142 A1 beschreibt ein zweikomponentiges Retraktionssystem, in dessen Zusammensetzung Silikate aus der Tonmineraliengruppe und der Glimmergruppe enthalten sind. Hierbei umfasst die Zusammensetzung eine Flüssigkeit und ein 1:1-Schichtsilikat und ein 2:1-Schichtsilikat in einem Verhältnis von 50/50 bis 5/95 wt% zueinander.
Ein Adstringens wird in dieser Zusammensetzung nicht zwingend beansprucht (Absatz [0123]). Falls jedoch adstringente Eigenschaften erwünscht sind, sind die folgenden Verbindungen offenbart: Oxide, Chloride oder Sulfate von Eisen (z.B. Eisensulfat, Eisensubsulfat, Eisenchlorid), Aluminium (z.B. Kaliumaluminiumsulfat, Aluminiumammoniumsulfat, Aluminiumsulfat, Aluminiumchlorohydrat, Aluminiumacetat) und Zink, Polyphenole, Ellagsäure, Permanganate (z.B. Kaliumpermanganat), Kaliumferrat (IV), Silbernitrat und Wasserstoffperoxid, Adrenalin und Mischungen dieser Verbindungen. Eine bevorzugte Klasse ist die Klasse der Aluminiumverbindungen.

US 2010/0248190 A1 beschreibt eine Retraktionspaste, umfassend ein polymerisierbares Monomer, einen Photoinitiator, ein feines anorganisches Pulver und ein Adstringens. Folgende Adstringentien werden beansprucht: Alaun, Aluminiumchlorid, Aluminiumsulfat, Zinkchlorid, Zinksulfat, Adrenalin, Tannine sowie Kombinationen dieser Verbindungen.

In WO 2006/057535 wird ein Retraktionsmaterial beschrieben, dessen Zusammensetzung Kaolin, destilliertes Wasser, Aluminiumchloridhexahydrat, Stärkepulver, Silikonöl und Farbstoff umfasst. Als Adstringens wird alternativ noch das Eisen(III)sulfat angegeben.

WO 2010/117442 A1 beansprucht eine Retraktionspaste, in der sowohl eine adstringierende also auch eine flüssigkeitsabsorbierende Substanz enthalten sind. Die hier beanspruchten Adstringentien sind das Aluminiumchlorid, Aluminiumsulfat, Eisenchlorid, Aluminiumkaliumsulfat, Ammoniumaluminiumsulfat und die Tanninsäure.

US 4,468,202 A beschreibt ein Verfahren zur dentalen Abformung, wobei ein chemisch- oder lichthärtendes elastomeres Material, zunächst zwischen Zahn und Zahnfleisch appliziert und gehärtet wird und dann ein weiteres elastomeres Material für die Abformung appliziert und ausgehärtet wird, sodass sich beide Materialien verbinden. Als elastomere Materialien, die in diesem Verfahren beansprucht werden, sind Polyester, Polyether, Polysulfide, Polysiloxane und Polyolefine benannt. Die in dem Verfahren verwendeten Zusammensetzungen können auch Adstringentien beinhalten. Konkrete Verbindungen werden nicht genannt.

In US 7,195,483 B2 wird eine Technik dargestellt, bei der man zunächst eine adstringierende Flüssigkeit auf den präparierten Zahn gibt, um die Blutung zu stoppen bzw. zu reduzieren. Als adstringierende Mittel werden beansprucht: Aluminiumkaliumsulfat, Kaliumaluminiumsulfat, Aluminiumsulfat, Alaun, Eisensulfat, Aluminiumammoniumsulfat, Eisenchlorid, Aluminiumchlorid, Natriumchlorid und Zinkchlorid.

WO 2016/196028 A1 gibt eine Methode an, in der zunächst ein Material, umfassend härtbare Komponenten und einen Katalysator in den Sulcus appliziert wird. In einem weiteren Schritt wird ein zweites Material mit einer geringeren Konsistenz auf den präparierten Zahn aufgetragen. Diese Materialien werden dann zusammen gehärtet und entfernt und somit hat man dann direkt die Abformung. Das verwendete Material ist entweder ein Silikonsystem mit härtbaren Siloxanendgruppen, gebunden an einem Dimethylsiloxangrundgerüst oder ein Polyethersystem mit härtbaren Aziridinendgruppen, gebunden an einem Polyethergrundgerüst. Die hier offenbarten Adstringentien umfassen Aluminiumsalze wie Aluminiumsulfat, Aluminiumammoniumsulfat, Aluminiumchlorhydrat, Aluminiumacetat und Mischungen dieser Verbindungen. Geeignete Adstringentien können auch Eisen-, Mangan und/oder Zinkverbindungen enthalten.

WO 2016/196048 A1 beschreibt ein System für die Retraktion und Abformung, umfassend drei Pasten, welche in verschiedenen Zusammensetzungen auf den Zahn appliziert werden können. Auch in diesem System können Adstringentien verwendet werden, konkrete Beispiele werden nicht genannt.

WO 2018/085744 A1 offenbart Retraktionszusammensetzungen auf Basis guanidinylhaltiger Polymere. Die Zusammensetzungen enthalten zudem Füllstoffe und Pastenbildner.

US 8,106,030 B2 offenbart ein hämostatisches Pulver zur Verwendung in der Herstellung eines Wundverbands zur Versorgung ernsthafter, lebensbedrohlicher Wunden, die sich beispielsweise Personen während eines Kriegseinsatzes zuziehen. Der Einsatz dieses Pulvers ist für den militärischen Bereich vorgesehen. Dentale Verwendungsmöglichkeiten werden nicht erwähnt. Dieser Wundverband enthält ein Chitosansalz, ein inertes Material und eine organische Säure. Genannt werden die Säuren Essigsäure, Milchsäure, Bernsteinsäure, Äpfelsäure, Schwefelsäure und Acrylsäure, wobei die Bernsteinsäure bevorzugt ist. Das Pulver besitzt natürlich keine retrahierenden Eigenschaften zum Verschieben der Gingiva.

In der Schrift WO 2016/164903 A1 wird ein Chitosan Gel zum Stillen von Blutungen offenbart, das eingesetzt wird für Stirnhöhleneingriffe, HNO-Behandlungen, orale - und gesichtschirurgische Eingriffe, für orthopädische und urologische Eingriffe, in der Wiederherstellungschirurgie, in der kosmetischen Chirurgie, in der Gefäßchirurgie, in der Transplantationschirurgie, bei neurologischen, onkologischen und gynäkologischen Eingriffen. In bestimmten Ausführungsformen werden in diesem Gel organische Säuren verwendet, wobei der Einsatz von monofunktionalen Säuren zwingend ist. Bevorzugt ist der Gebrauch von Milchsäure, wobei die Zugabe von difunktionellen oder trifunktionellen Säuren optional ist. Das hier offenbarte Gel weist keine retrahierenden Eigenschaften zum Verschieben der Gingiva auf.

WO 2009/061409 A1 beschreibt stabile alkoholische Chitosan Lösungen, die antivirale, antibakterielle und hämostatische Eigenschaften vorweisen. Auch sie sind nicht zum Verschieben der Gingiva geeignet.

EP 2 392 310 A1 beschreibt eine photopolymerisierbare Zusammensetzung zur temporären Sulcuserweiterung. Als adstringiernde Bestandteile werden Alaune, Aluminiumchlorid, Aluminiumsulfat, Zinkchlorid, Zinksulfat, Epinephrin sowie Tannin beansprucht.

EP 2 022 464 A2 beschreibt die Verwendung einer photopolymerisierbaren Retraktionszusammensetzung zur temporären Sulcuserweiterung. Als adstringiernde Bestandteile der Zusammensetzung werden Alaune, Aluminiumchlorid, Aluminiumsulfat, Eisenchlorid, Eisensulfat, Zinkchlorid, Zinksulfat sowie Epinephrin beansprucht.

In US 2018/0064844 A1 wird eine hämostatische, durch Licht polymerisierbare Retraktionspaste angegeben. Diese Retraktionspaste wird vom Präparationsrand entfernt, indem sie nach dem Härten herausgezogen wird. Nachteilig hierbei ist, dass beim Herausnehmen des gehärteten Materials, Stücke verschiedener Größen im Sulcus verbleiben können und somit Entzündungen hervorrufen können. Außerdem kann das hämostatische Mittel, durch die starre Struktur des Polymerisats nicht effektiv freigesetzt werden.

US 2005/0008583 A1 beschreibt eine Retraktionspaste aus Kaolin, Aluminiumchlorid und Wasser/Glycerin.

Aufgabe der vorliegenden Erfindung ist es somit, eine Retraktionspaste anzugeben, die eine genauso gute Retraktion und adstringierende Wirkung vorweist wie die Standardzusammensetzungen mit Aluminiumsalzen aus dem Stand der Technik, die aber durch ihre Zusammensetzung nicht gesundheitsschädlich ist.

Unter aluminiumfrei wird im Rahmen der vorliegenden Erfindung verstanden, dass die Zusammensetzung keine löslichen Aluminiumsalze, wie z.B. Aluminiumchlorid, Aluminiumsulfat, Kaliumaluminiumsulfat, Aluminiumammoniumsulfat, Aluminiumchlorhydrat, Aluminiumacetat enthält. Kaolin oder andere mineralische Füllstoffe, die Aluminium fest im Atomverbund des Minerals gebunden haben und es unter den Bedingungen der Anwendung nicht freisetzen können, fallen jedoch in den Begriff "aluminiumfrei".

Als Alternative zu Aluminiumsalzen wäre denkbar, eine Retraktionspaste, die Chitosan bzw. Chitosanderivate enthält, zu verwenden, da Chitosan nicht gesundheitsschädlich ist und durch die Proteinkoagulation adstringierend wirkt.

Chitosan, ein Polyaminosaccharid, wird hauptsächlich aus Chitin, welches unter anderem in Krustentieren enthalten ist, durch eine Deacetylierung gewonnen. Es gibt Chitosan mit verschiedenen Molekulargewichten von ca. 10.000 bis 2.000.000 Da. Wegen seiner besonderen Eigenschaften, beispielsweise hohe Lipid- und Proteinbindung, hohe Flüssigkeitsbindung und Atoxizität wird Chitosan in einer Vielzahl von Bereichen eingesetzt. Unter Anderem findet Chitosan seinen Einsatz in der Medizin, Kosmetikindustrie und auch Lebensmittelindustrie. Für Retraktionszusammensetzungen erscheint die chitosaninduzierte Proteinkoagulation interessant. Der Einsatz von Chitosan in adstringierenden Zusammensetzungen ist bereits bekannt. So gibt es beispielsweise Kompressen, die durch das enthaltene Chitosan zur Stillung stark blutender Wunden benutzt werden.

Um die gewünschte adstringierende Wirkung von Chitosan zu erreichen, wird es in verdünnter Säure gelöst. Durch die Protonierung der Aminogruppen kann das Chitosan Proteine binden, sodass eine Koagulation bewirkt wird.

Überraschenderweise zeigte sich, dass die Koagulation von Proteinen und somit eine adstringierende Wirkung nur in Kombination einer bestimmten Säure mit Chitosan bzw. Chitosanderivaten auftritt. Mit Chitosanderivaten sind beispielsweise Chitosanhydrochlorid, Chitosan Glutamat, Chitosan Lactat, Chitosan Acetat und Carboxymethylchitosan gemeint. Besonders geeignet sind Chitosan oder Chitosanderivate mit einem Molekulargewicht von 1.000 bis 800.000 Da, vorzugsweise von 5.000 bis 500.000 Da, besonders bevorzugt von 10.000 bis 300.000 Da. Besonders geeignet sind zudem Chitosan oder Chitosanderivate mit einem Deacetylierungsgrad von 60 bis 100%, vorzugsweise von 75 bis 100%, besonders bevorzugt von 90 bis 100%.

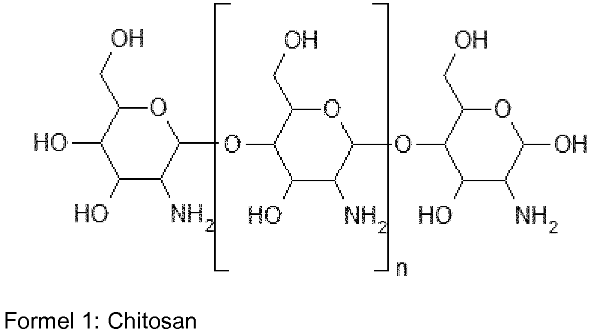

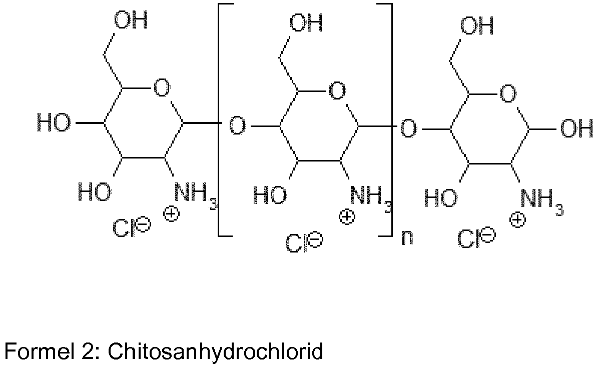

Standardmäßig wird Chitosan in Essigsäure verdünnt, allerdings funktioniert der adstringierende Mechanismus nicht mit Essigsäure.

Zitronensäure bzw. Citrat wirkt antikoagulierend und wird daher als Alternative zu Heparin bei der extrakorporalen Blutreinigung eingesetzt [EP 2 200 675 B1; Janssen MJ et al. (1993), Nephrol Dial Transplant, 8 (11): 1228-1233; Pinnick et al. (1983), N Engl J Med, Vol. 308, 258-261]. Die antikoagulierende Wirkung des Citrats beruht auf dessen Komplexbildung mit freien zweiwertigen Calciumionen. Ionisiertes Calcium ist ein wichtiger Cofaktor im Gerinnungssystem, da es eine katalytische Wirkung auf die meisten Enzyme der Gerinnungskaskade hat. Eine Komplexierung der Calciumionen durch Citrat führt folglich zu einer Unterbindung des Ablaufs der Gerinnungskaskade.

Ebenso wie Citrat wirkt Oxalat calciumkomplexierend und antikoagulierend. Beide werden daher zur Blutplasmaherstellung bei der aPTT (activated Partial Thromboplastin Time) eingesetzt.

Es ist daher völlig überraschend, dass Weinsäure, die ebenfalls Calcium bindet, in Kombination mit Chitosan dessen koagulierende Eigenschaft bewirkt.

Vorteilhaft gegenüber US 2018/0064844 A1 ist es, dass sich die erfindungsgemäße Retraktionspaste einfach und rückstandslos mit Wasser entfernen lässt und das adstringierende Chitosan bzw. die Chitosanderivate besser freigesetzt werden können und somit ihre Wirkung direkt entfalten können. Erfindungsgemäße adstringierende Retraktionspasten enthalten ein Lösungsmittel. Bevorzugt werden als Lösungsmittel Wasser und/oder Alkohole eingesetzt.

Als Alkohole können beispielsweise Ethanol, Propanol, Butanol, sowie die mehrwertigen Alkohole Ethylenglykol, Propylenglykol, Butylenglykol, Glyzerin, Trimethylolpropan, Pentaerythritol, Di-Pentaerythritol sowie die Polyalkylenglykole Diethylenglykol, Triethylenglykol, Tetraethylenglykol, Polyethylenglykol, Dipropylenglykol, Tripropylenglykol, Tetrapropylenglykol, Polypropylenglykol eingesetzt werden.

In einer bevorzugten Ausführungsform umfasst das Lösungsmittel der adstringierenden Retraktionspaste Wasser oder besteht daraus. Erfindungsgemäße Pastenzusammensetzungen enthalten bevorzugt anorganische und/oder organische Füllstoffe.

Als organische Füllstoffe können Polyethylenpulver, Polypropylenpulver, PMMA-Pulver, Polyurethanpulver sowie Splitter oder Perlpolymerisate eingesetzt werden. Als anorganische Füllstoffe können Silikate, Schichtsilikate, Cristobalit, Quarz, pyrogene Kieselsäure, Wollastonit, Dentalgläser, Titandioxid, Seltenerdoxide und -fluoride, Bariumsulfat, Zirkonoxid oder Magnesiumchlorid eingesetzt werden.

Tone sind natürlich vorkommende feinkörnige Partikel, die aus einer Vielzahl von siliziumreichen Phyllosilikatmineralien in Form von Oxiden und Hydroxiden mit unterschiedlich viel Kristallwasser bestehen. Bekannte Gruppen von Tonmineralien sind: Kaolinit, Smektit, Illit und Chlorit. Kaolinite sind die Mineralien Kaolinit, Dickit, Halloysit und Ancrite. Zu den Smektiten gehören Pyrophyllit, Talkum, Vermiculit, Sauconit, Saponit, Nontronit und Montmorillonit. Zu den Illiten gehört Glimmer. Chlorite enthalten eine Vielzahl von ähnlichen Mineralien mit erheblichen chemischen Schwankungen. Kaolinit- und Smektit-Tone werden für Hautpflegeanwendungen eingesetzt. Montmorillonit ist ein sehr weiches Mineral aus der Gruppe der Smektite. Bentonit ist ein Ton, der hauptsächlich aus Montmorillonit besteht. Bentonit und Montmorillonit werden manchmal austauschbar verwendet, um sich auf das gleiche Mineral zu beziehen. Es existieren zwei Arten von Bentonit: Natriumbentonit (Quellbentonit) und Calciumbentonit (nicht quellender Bentonit). Bentonite entstehen aus der hydrothermalen Verwitterung von Vulkanasche.

Der Ton kann ein Schichtton sein, der Kaolinit, Montmorillonit (Bentonit), Talkum, Glimmer (Illit), Serpentin, Chlorit, Mullit, Kyanit, Bimsstein, Goethit und/oder Pyrophyllit enthält.

Bevorzugt werden Kaolin oder Magnesiumchlorid jeweils alleine oder in Kombination mit pyrogenen Kieselsäuren eingesetzt.

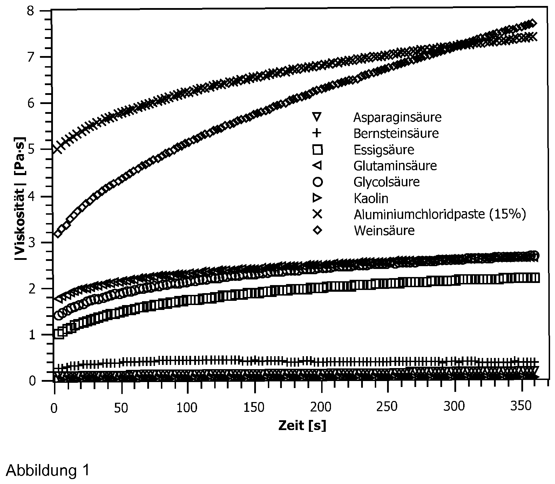

Kaolin selbst kann als oberflächenaktive Substanz ebenfalls koagulierend wirken. Es wird daher auch in den Gerinnungstests aPTT (activated Partial Thromboplastin Time) und ACT (Activated Coagulation Time) eingesetzt. Es absorbiert Wasser und führt so zu einer Konzentration der Gerinnungsfaktoren. Diese Wirkungsweise steht jedoch in einer bereits wässrigen Zusammensetzung nicht mehr zur Verfügung (siehe Abbildung 1).

Bevorzugt ist eine adstringierende Retraktionspaste für den professionellen zahnärztlichen Gebrauch, umfassend
a.) 0,1 bis 30 Gew.-%, vorzugsweise 0,5 bis 20 Gew.-%, besonders bevorzugt 1 bis 10 Gew.-% Chitosan und/oder Chitosanderivate,
b.) 0,1 bis 30 Gew.-%, vorzugsweise 0,5 bis 20 Gew.-%, besonders bevorzugt 1 bis 15 Gew.-% Weinsäure,
c.) 20 bis 80 Gew.-%, vorzugsweise 30 bis 75 Gew.-%, besonders bevorzugt 45 bis 75 Gew.-% Lösungsmittel,
d.) 1 bis 75 Gew.-%, vorzugsweise 5 bis 60 Gew.-%, besonders bevorzugt 5 bis 55 Gew.-% Füllstoffe,
wobei sich die Gew.-%-Angaben auf die Gesamtzusammensetzung beziehen.

Vorteilhaft wird eine erfindungsgemäße adstringierende Retraktionspaste in einem therapeutischen Verfahren zur Restauration eines oder mehrerer Zähne angewendet.

Die vorliegende Erfindung betrifft somit auch ein Kit zur Durchführung der Abformung im Rahmen der Restauration. Ein solches Kit umfasst
- eine erfindungsgemäße adstringierende Pastenzusammensetzung,
- ein Abformmaterial, vorzugsweise ein Silikon- oder Polyetherabformmaterial,
- sowie optional einen konfektionierten oder individuellen Löffel.

### Ausführungsbeispiele der Erfindung

### Bestimmung der Koagulation

Die Bestimmung der Proteinkoagulation wurde an der Modellsubstanz "Milch" durchgeführt. Dieses Verfahren ist in der Wissenschaft seit vielen Jahren bekannt (siehe beispielsweise A. Narracott et al., "Development and validation of models fort he investigation of blood clotting in idealized stenoses and cerebral aneurysms", J. Artif. Organs (2005), 8, 56 - 62 oder L.A. Keggen et al. "The use of enzyme activated milk for in vitro simulation of prosthetic valve thrombosis", J. Heart Valve Dis., (1996), 5, 74 - 83).

Die Milchproteinkoagulation wurde in unserer Testserie zunächst qualitativ visuell bewertet. Dazu wurden zunächst 1,5 ml Milch (3,5% H-Milch) in eine Eppendorf Tube gefüllt und 200 mg der Paste in 1 ml demineralisiertem Wasser suspendiert. Der Milch wurden 0,5 ml von der Suspension hinzugefügt und kurz geschüttelt. Die Bewertung "positiv" erfolgte, wenn innerhalb von 180 Sekunden nach Schütteln eine deutliche Ausflockung erfolgte. Die Bewertung "negativ" erfolgte, wenn das Aussehen und die Konsistenz der Suspension 180 Sekunden nach Schütteln unverändert erschienen.

Die Bestimmung der Proteinkoagulation wurde zudem quantitativ viskosimetrisch an der Modellsubstanz "Milch" durchgeführt. Für die Bestimmung der Koagulation wurden zunächst 1,5 ml Milch (3,5% H-Milch) in eine Eppendorf Tube gefüllt und 200 mg der Paste in 1 ml demineralisiertem Wasser suspendiert. Der Milch wurden 0,5 ml von der Suspension hinzugefügt. Nach einem kurzen Schütteln wurde die Mischung sofort mit der Pipette auf das Rheometer MCR 301 (Fa. Anton Paar) überführt und die Messung gestartet. Für die Messung wurde das Rheometer mit einer Frequenz von 4 Hz, 1% Amplitude bei einer Temperatur von 23°C betrieben.

Tabelle 2 zeigt die Resultate unserer Untersuchung zur Proteinkoagulation. Die Ergebnisse der viskosimetrischen Messung können als Maß für den Grad der Ausflockung und somit als quantitative Angabe für die Koagulationsneigung herangezogen werden.

Mit Blutplasma wurden die Resultate, die an Milch gewonnen wurden, verifiziert. Blutplasma ist kommerziell erhältlich (z.B. Pooled Normal Plasma - George King Bio-Medical, Inc.). Dazu wurden zunächst 1,5 ml Blutplasma in eine Eppendorf Tube gefüllt und 200 mg der Paste in 1 ml demineralisiertem Wasser suspendiert. Dem Blutplasma wurden 0,5 ml von der Suspension hinzugefügt und kurz geschüttelt. Die Bewertung "positiv" erfolgte, wenn innerhalb von 180 Sekunden nach Schütteln eine deutliche Koagulation erfolgte. Die Bewertung "negativ" erfolgte, wenn das Aussehen und die Konsistenz der Suspension 180 Sekunden nach Schütteln unverändert erschienen.

### Beispiel

Für die Herstellung der Retraktionspasten mischt man zunächst Chitosan mit einer wässrigen Säurelösung, wodurch eine Lösung entsteht. Damit daraus eine Paste hergestellt werden kann, wird diese Lösung dann schrittweise mit einem Füllstoff versetzt. In der folgenden Tabelle sind neun Beispiele für die Zusammensetzung einer Retraktionspaste angegeben.

**Tabelle 1:**

| | **Zusammensetzung** | | | | | | | | |
|---|---|---|---|---|---|---|---|---|---|
| | **1** | **2** | **3** | **4** | **5** | **6** | **7** | **8** | **9** |
| **Inhaltsstoffe** | **m%** | **m%** | **m%** | **m%** | **m%** | **m%** | **m%** | **m%** | **m%** |
| Chitosan | 1 | 1 | 1 | 2 | 2 | 2 | 4 | 4 | 8 |
| Weinsäure | 1 | 1 | 1 | 3 | 3 | 3 | 6 | 6 | 11 |
| Wasser | 48 | 65 | 78 | 50 | 67 | 77 | 50 | 66 | 72 |
| Kaolin | 50 | 20 | - | 45 | 18 | - | 40 | 16 | - |
| MgCl₂ | - | - | 16 | - | - | 15 | - | - | 8 |
| Aerosil 200 | - | 13 | 4 | - | 10 | 3 | - | 8 | 1 |
| **Summe** | **100** | **100** | **100** | **100** | **100** | **100** | **100** | **100** | **100** |
| **Koagulation Chitosan-Paste (Milch)** | positiv | positiv | positiv | positiv | positiv | positiv | positiv | positiv | positiv |
| **Koagulation Chitosan-Paste (Blutplasma)** | positiv | positiv | positiv | positiv | positiv | positiv | positiv | positiv | positiv |

Im Folgenden wurde ausgehend von Paste 7 der Einfluss unterschiedlicher organischer Säuren untersucht. Die Zusammensetzungen entsprechen der Zusammensetzung 7, wobei statt Weinsäure jeweils die in der Tabelle 2 angegebenen organischen Säuren Essigsäure, Glycolsäure, Bernsteinsäure, Asparaginsäure, Glutaminsäure, Zitronensäure bzw. Malonsäure verwendet wurden.

Entsprechende Zusammensetzungen mit Milchsäure und Ascorbinsäure erwiesen sich als nicht stabil.

**Tabelle 2:**

| **Säure/Base** | **pKs** | **Koagulation Chitosan-Paste (Milch)** | **Koagulation Chitosan-Paste (Blutplasma)** | **Viskosität (Pa·s) nach 360 Sekunden** |
|---|---|---|---|---|
| Weinsäure (Bsp. 7) | 2,98/4,34 | positiv | positiv | 7,68 |
| Essigsäure | 4,76 | negativ | negativ | 2,19 |
| Glycolsäure | 3,83 | negativ | negativ | 2,66 |
| Bernsteinsäure | 4,16 / 5,61 | negativ | negativ | 0,35 |
| Asparaqinsäure | 1,99 / 3,9 | negativ | negativ | 0,16 |
| Glutaminsäure | 2,16 / 4,32 / 9,96 | negativ | negativ | 2,62 |
| Zitronensäure | 3,13 / 4,76 / 6,4 | negativ | negativ | |
| Malonsäure | 2,83 / 5,69 | negativ | negativ | |
| Aluminiumchloridpaste (15%) | | positiv | positiv | 7,38 |

Beispiel 7 ist erfindungsgemäß; alle anderen Beispiele in Tabelle 2 sind Vergleichsbeispiele.

In Abbildung 1 sind beispielhaft die Messkurven für Weinsäure, Glycolsäure, Essigsäure, Glutaminsäure, Asparaginsäure, Bernsteinsäure und Kaolin dargestellt. Hierbei dient die Blindprobe mit Kaolin als Referenzwert.

Als Standardzusammensetzung aus dem Stand der Technik wurde zum Vergleich mit der erfindungsgemäßen aluminiumfreien Paste die aluminiumhaltige Retraktionspaste Expasyl (15 Gew.-% Aluminiumchlorid) der Firma Acteon verwendet.

## Patentansprüche

1. Adstringierende Retraktionspaste für den professionellen zahnärztlichen Gebrauch, umfassend
a.) Chitosan und/oder Chitosanderivate,
b.) Weinsäure,
c.) Lösungsmittel und
d.) Füllstoff.

2. Adstringierende Retraktionspaste für den professionellen zahnärztlichen Gebrauch nach Anspruch 1, wobei das Lösungsmittel ausgewählt wird, aus der Gruppe umfassend: Wasser und Alkohole.

3. Adstringierende Retraktionspaste für den professionellen zahnärztlichen Gebrauch nach Anspruch 2, wobei der Alkohol ausgewählt ist aus der Gruppe bestehend aus Ethanol, Propanol, Butanol, sowie die mehrwertigen Alkohole Ethylenglykol, Propylenglykol, Butylenglykol, Glyzerin, Trimethylolpropan, Pentaerythritol, Di-Pentaerythritol sowie die Polyalkylenglykole Diethylenglykol, Triethylenglykol, Tetraethylenglykol, Polyethylenglykol, Dipropylenglykol, Tripropylenglykol, Tetrapropylenglykol und Polypropylenglykol.

4. Adstringierende Retraktionspaste für den professionellen zahnärztlichen Gebrauch nach Anspruch 1 oder 2, wobei das Lösungsmittel Wasser umfasst oder aus Wasser besteht.

5. Adstringierende Retraktionspaste für den professionellen zahnärztlichen Gebrauch nach einem der vorangehenden Ansprüche, wobei das Chitosan oder das Chiotosanderivat ein Molekulargewicht von 1.000 bis 800.000 Da, vorzugsweise von 5.000 bis 500.000 und besonders bevorzugt von 10.000 bis 300.000 aufweist.

6. Adstringierende Retraktionspaste für den professionellen zahnärztlichen Gebrauch nach einem der vorangehenden Ansprüche, wobei das Chitosan oder das Chitosanderivat einen Deacetylierungsgrad von 60 bis 100%, vorzugsweise von 75 bis 100% und besonders bevorzugt von 90 bis 100% aufweist.

7. Adstringierende Retraktionspaste für den professionellen zahnärztlichen Gebrauch nach einem der vorangehenden Ansprüche, wobei der Füllstoff ein anorganischer und/oder organischer Füllstoff ist.

8. Adstringierende Retraktionspaste für den professionellen zahnärztlichen Gebrauch nach einem der Vorangehenden Ansprüche, wobei der Füllstoff Kaolin und/oder Magnesiumchlorid umfasst.

9. Adstringierende Retraktionspaste für den professionellen zahnärztlichen Gebrauch nach einem der vorangehenden Ansprüche, umfassend
a.) 0,1 bis 30 Gew.-%, vorzugsweise 0,5 bis 20 Gew.-%, besonders bevorzugt 1 bis 10 Gew.-% Chitosan und/oder Chitosanderivate,
b.) 0,1 bis 30 Gew.-%, vorzugsweise 0,5 bis 20 Gew.-%, besonders bevorzugt 1 bis 15 Gew.-% Weinsäure,
c.) 20 bis 80 Gew.-%, vorzugsweise 30 bis 75 Gew.-%, besonders bevorzugt 45 bis 75 Gew.-% Lösungsmittel,
d.) 1 bis 75 Gew.-%, vorzugsweise 5 bis 60 Gew.-%, besonders bevorzugt 5 bis 55 Gew.-% Füllstoffe,
wobei sich die Gew.-%-Angaben auf die Gesamtzusammensetzung beziehen.

10. Adstringierende Retraktionspaste nach einem der vorangehenden Ansprüche zur Anwendung in einem therapeutischen Verfahren zur Restauration eines oder mehrerer Zähne.

11. Kit umfassend
- eine adstringierende Pastenzusammensetzung nach einem der Ansprüche 1 bis 9,
- ein Abformmaterial, vorzugsweise ein Silikon- oder Polyetherabformmaterial,
- optional einen konfektionierten oder individuellen Löffel.

## Claims

1. Astringent retraction paste for professional dental use, comprising
a.) chitosan and/or chitosan derivatives,
b.) tartaric acid,
c.) solvent and
d.) filler.

2. Astringent retraction paste for professional dental use according to Claim 1, wherein the solvent is selected from the group encompassing the following: water and alcohols.

3. Astringent retraction paste for professional dental use according to Claim 2, wherein the alcohol is selected from the group consisting of ethanol, propanol and butanol and also the polyhydric alcohols ethylene glycol, propylene glycol, butylene glycol, glycerol, trimethylolpropane, pentaerythritol and dipentaerythritol and also the polyalkylene glycols diethylene glycol, triethylene glycol, tetraethylene glycol, polyethylene glycol, dipropylene glycol, tripropylene glycol, tetrapropylene glycol and polypropylene glycol.

4. Astringent retraction paste for professional dental use according to Claim 1 or 2, wherein the solvent comprises water or consists of water.

5. Astringent retraction paste for professional dental use according to any of the preceding claims, wherein the chitosan or the chitosan derivative has a molecular weight of 1000 to 800 000 Da, preferably from 5000 to 500 000 and more preferably from 10 000 to 300 000.

6. Astringent retraction paste for professional dental use according to any of the preceding claims, wherein the chitosan or the chitosan derivative has a degree of deacetylation of 60% to 100%, preferably of 75% to 100% and more preferably of 90% to 100%.

7. Astringent retraction paste for professional dental use according to any of the preceding claims, wherein the filler is an inorganic and/or organic filler.

8. Astringent retraction paste for professional dental use according to any of the preceding claims, wherein the filler comprises kaolin and/or magnesium chloride.

9. Astringent retraction paste for professional dental use according to any of the preceding claims, comprising
a.) 0.1 to 30 wt%, preferably 0.5 to 20 wt%, more preferably 1 to 10 wt% of chitosan and/or chitosan derivatives,
b.) 0.1 to 30 wt%, preferably 0.5 to 20 wt%, more preferably 1 to 15 wt% of tartaric acid,
c.) 20 to 80 wt%, preferably 30 to 75 wt%, more preferably 45 to 75 wt% of solvent,
d.) 1 to 75 wt%, preferably 5 to 60 wt%, more preferably 5 to 55 wt% of fillers,
the wt% figures being based on the overall composition.

10. Astringent retraction paste according to any of the preceding claims for use in a therapeutic method for restoring one or more teeth.

11. Kit comprising
- astringent paste composition according to any of Claims 1 to 9,
- an impression material, preferably a silicone or polyether impression material,
- optionally a prefabricated or individual tray.

## Revendications

1. Pâte de rétraction astringente pour l'utilisation dentaire professionnelle, comprenant
a.) du chitosane et/ou un dérivé de chitosane,
b.) de l'acide tartrique,
c.) un solvant et
d.) une charge.

2. Pâte de rétraction astringente pour l'utilisation dentaire professionnelle selon la revendication 1, le solvant étant choisi dans le groupe comprenant : l'eau et les alcools.

3. Pâte de rétraction astringente pour l'utilisation dentaire professionnelle selon la revendication 2, l'alcool étant choisi dans le groupe constitué par l'éthanol, le propanol, le butanol, ainsi que les alcools polyvalents éthylèneglycol, propylèneglycol, butylèneglycol, glycérine, triméthylolpropane, pentaérythritol, le dipentaérythritol ainsi que les polyalkylèneglycols diéthylèneglycol, triéthylèneglycol, tétraéthylèneglycol, polyéthylèneglycol, dipropylèneglycol, tripropylèneglycol, tétrapropylèneglycol et polypropylèneglycol.

4. Pâte de rétraction astringente pour l'utilisation dentaire professionnelle selon la revendication 1 ou 2, le solvant comprenant de l'eau ou étant constitué d'eau.

5. Pâte de rétraction astringente pour l'utilisation dentaire professionnelle selon l'une quelconque des revendications précédentes, le chitosane ou le dérivé de chitosane présentant un poids moléculaire de 1 000 à 800 000 Da, de préférence de 5 000 à 500 000 et particulièrement préférablement de 10 000 à 300 000.

6. Pâte de rétraction astringente pour l'utilisation dentaire professionnelle selon l'une quelconque des revendications précédentes, le chitosane ou le dérivé de chitosane présentant un degré de déacétylation de 60 à 100 %, de préférence de 75 à 100 % et particulièrement préférablement de 90 à 100 %.

7. Pâte de rétraction astringente pour l'utilisation dentaire professionnelle selon l'une quelconque des revendications précédentes, la charge étant une charge inorganique et/ou organique.

8. Pâte de rétraction astringente pour l'utilisation dentaire professionnelle selon l'une quelconque des revendications précédentes, la charge comprenant du kaolin et/ou du chlorure de magnésium.

9. Pâte de rétraction astringente pour l'utilisation dentaire professionnelle selon l'une quelconque des revendications précédentes, comprenant
a.) 0,1 à 30 % en poids, de préférence 0,5 à 20 % en poids, particulièrement préférablement 1 à 10 % en poids de chitosane et/ou de dérivés de chitosane,
b.) 0,1 à 30 % en poids, de préférence 0,5 à 20 % en poids, particulièrement préférablement 1 à 15 % en poids d'acide tartrique,
c.) 20 à 80 % en poids, de préférence 30 à 75 % en poids, particulièrement préférablement 45 à 75 % en poids de solvant,
d.) 1 à 75 % en poids, de préférence 5 à 60 % en poids, particulièrement préférablement 5 à 55 % en poids de charges,
les données en % en poids se rapportant à la composition totale.

10. Pâte de rétraction astringente selon l'une quelconque des revendications précédentes pour l'application dans un procédé thérapeutique pour la restauration d'une ou plusieurs dents.

11. Kit comprenant
- une composition de pâte astringente selon l'une quelconque des revendications 1 à 9,
- un matériau d'empreinte, de préférence un matériau d'empreinte de silicone ou de polyéther,
- éventuellement une cuillère confectionnée ou individuelle.
